# EUROPÄISCHE PATENTANMELDUNG

(11) **EP 0 538 758 A1**
(43) Veröffentlichungstag der Anmeldung: **28.04.1993**
(21) Anmeldenummer: 92117787.9
(22) Anmeldetag: 17.10.1992
(51) Int. Cl.: A61F 13/04

(54) **Gehsohle**

(30) Priorität: 24.10.1991 DE 4135065
(71) Anmelder: Elastogran GmbH, D-49440 Lemförde (DE)
(72) Erfinder: Hoehner, Peter, W-2844 Stemshorn (DE); Adler, Lothar, W-4500 Osnabrueck (DE); Bloemer, Alois, Prof. Dr., W-4390 Gladbeck (DE)
(74) Vertreter: Langfinger, Klaus-Dieter, Dr.

(57) **Zusammenfassung**

Die Gehsohle für einen der Ruhigstellung und/oder der Stützung dienenden Unter- und/oder Oberschenkelverband besitzt einen Sohlenkörper (1) mit einer Oberseite als Aufstandsfläche für den Fuß und einer Unterseite als Lauffläche. Auf die Oberseite und/oder Unterseite des Sohlenkörpers ist eine Auflage (5) aus elastischem Material aufgebracht und mit dem Sohlenkörper form- und/oder kraftschlüssig verbunden. Durch die Auflage läßt sich die Gehsohle insgesamt elastisch gestalten, so daß sich ein "weicher" Gang für den Patienten und damit optimale Heilungschancen ergeben.

## Beschreibung

Die Erfindung betrifft eine Gehsohle für einen der Ruhigstellung und/oder Stützung dienenden Unter- und/oder Oberschenkelverband, welche aus einem Sohlenkörper besteht, der eine für den Fuß als Aufstandsfläche dienende Oberseite und eine als Lauffläche dienende Unterseite aufweist, und mit Durchtrittsöffnungen für ein Befestigungsband oder dgl. oder für mehrere Befestigungsbänder oder dgl. ausgerüstet ist.

Eine derartige Gehsohle ist bekannt durch die DE 27 40 400 C2. Diese bekannte Gehsohle besteht im wesentlichen aus einem die erforderliche Festigkeit und Steifheit aufweisenden Sohlenkörper, insbesondere aus verschäumtem Polyurethan. Die Anlegung der Gehsohle erfolgt mittels Gurtbandsystemen, die Öffnungen in den Rand- und Fersenbereichen des Sohlenkörpers durchsetzen. Diese bekannte Gehsohle hat sich an sich gut bewährt, jedoch ergibt sich infolge der Festigkeit und Steifheit von deren Sohlenkörper ein in gewissem Maße "harter" Gang, welcher sich u.U. negativ auf den Heilungsverlauf auswirken kann.

Von diesem Stand der Technik ausgehend liegt der Erfindung die Aufgabe zugrunde, unter Vermeidung vorerwähnter Nachteile eine gattungsgemäße Gehsohle dahingehend zu verbessern, daß diese einen "weichen" Gang ermöglicht.

Zur Lösung dieser Aufgabe werden erfindungsgemäß die im Kennzeichen der Hauptansprüche aufgeführten Merkmale vorgeschlagen. Die zur vorteilhaften Ausgestaltung der Erfindung dienenden Mittel und Merkmale sind Gegenstand der Unteransprüche.

Die Erfindung folgt dem Leitgedanken, eine derartige Gehsohle "elastisch" zu gestalten, wodurch sich ein weicher Gang für den Patienten und damit optimale Heilungschancen ergeben.

Ein Ausführungsbeispiel der Erfindung ist an Hand der Zeichnung näher erläutert.

Es zeigt
- Figur 1: eine Seitenansicht einer Gehsohle,
- Figur 2: eine Draufsicht der Figur 1,
- Figur 3: eine geschnittene Seitenansicht der Figur 2 gemäß der Linie "C-C",
- Figur 4: eine geschnittene Stirnansicht der Figur 1 gemäß der Linie "B-B",
- Figur 5: eine geschnittene Stirnansicht der Figur 1 gemäß der Linie "A-A" und
- Figur 6: eine geschnittene Stirnansicht der Figur 2 gemäß der Linie "D-D".

Mit 1 ist der Sohlenkörper der Gehsohle bezeichnet, welcher in an sich bekannter Weise aus Polyurethan-Schaum hergestellt ist. Am rückwärtigen Ende des Sohlenkörpers 1 ist eine aufwärts gerichtete Stütze 2 als Anschlag für die Ferse eines Fußes vorgesehen. Die Ränder 3 des Sohlenkörpers 1 und der Stütze 2 sind im Sinne der Erzielung eines seitlichen Haltes des Fußes vorragend ausgebildet. In den vorragenden Rändern 3 sind Durchtrittsöffnungen 4 für nicht weiter dargestellte Gurtbänder vorgesehen, welche der Befestigung der Gehsohle am Fuß dienen.

Erfindungsgemäß ist auf die Oberseite des Sohlenkörpers 1 eine Auflage 5 aus elastischem Material aufgebracht, welche im dargestellten Falle die Oberseite vollständig abdeckt. Es ist jedoch auch möglich, diese Auflage 5 abschnittsweise auf der Oberseite des Sohlenkörpers vorzusehen. Als elastisches Material findet vorzugsweise ein zelliges Polyuethan-Elastomer Verwendung. Aufgrund der Auflage 5 aus elastischem Material ergibt sich ein federnder und definiert gedämpfter Gang für den Patienten, was sich vorteilhaft auf den Heilungsprozeß auswirkt. Vorteilhaft ist die Auflage 5 an der Oberseite des Sohlenkörpers 1 durch Kraftschluß und/oder Formschluß befestigt, beispielsweise durch Klebung, Heftung, Einschäumen, Hinterschäumen oder dgl.

In weiterer Ausgestaltung der Erfindung weist der Sohlenkörper 1 Durchtrittsöffnungen 6 auf, welche von Stollen 7 durchgesetzt sind. Vorteilhaft bestehen die Stollen 7 ebenfalls aus demselben elastischen Material wie die Auflage 5. Im dargestellten Ausführungsbeispiel sind die Auflage 5 und die Stollen 7 einstückig ausgeführt. Die Montage geschieht in der Weise, daß die Stollen 7 in die Durchtrittsöffnungen 6 des Sohlenkörpers "eingeknüpft" werden. Die Stollen 7 ergeben kleinflächige Federpunkte sowohl im Anroll-, als auch im Stand- und Abrollbereich der Gehsohle, so daß der gesamte Fußbereich definiert gefedert und gedämpft wird.

Es ist jedoch auch möglich, die Stollen 7 an die Auflage 5 durch die Durchtrittsöffnungen 6 des Sohlenkörpers hindurch anschäumen.

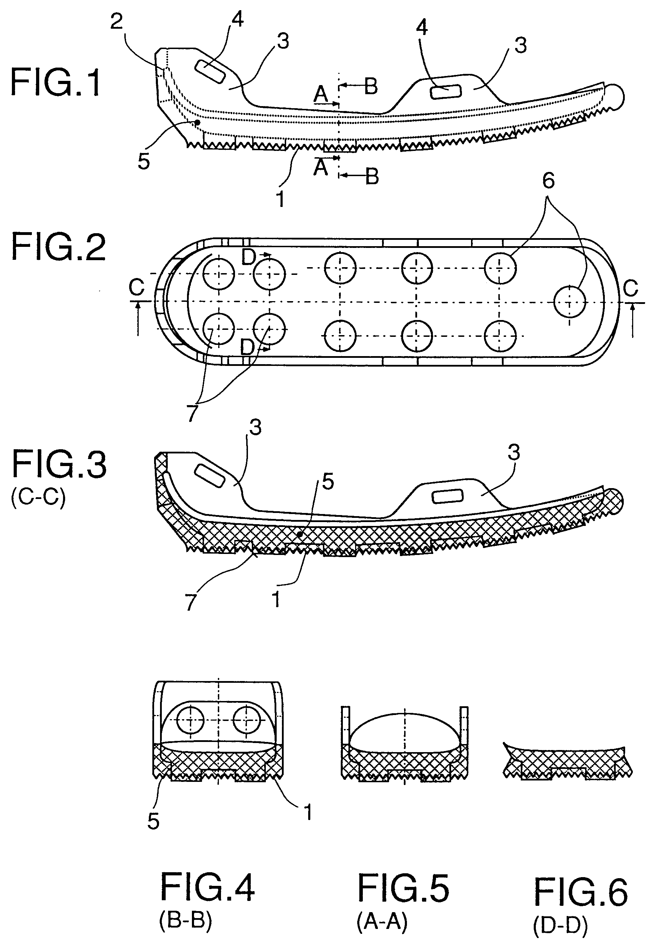

Wie insbesondere aus Figur 5 und 6 ersichtlich, erweitern sich die Durchtrittsöffnungen 6 des Sohlenkörpers 1 zu dessen Unterseite hin konisch, wobei die diesen zugeordneten Stollen 7 entsprechend gestaltet sind. Auf diese Weise ergibt sich eine einwandfreie Festlegung der Auflage 5 und der Stollen 7 am Sohlenkörper 1.

Nach einem weiteren Merkmal der Erfindung ragen die Stollen 7 über die Durchtrittsöffnungen 6 des Sohlenkörpers 1 über dessen Unterseite geringfügig vor, so daß sich außer dem angestrebten weichen Gang auch noch eine vergleichsweise hohe Rutschfestigkeit der Gehsohle ergibt.

Die Durchtrittsöffnungen 6 können über die Länge und/oder Breite des Sohlenkörpers 1 gleichmäßig verteilt angeordnet sein. Es ist jedoch auch möglich, diese nur in bestimmten Bereichen er Gehsohle vorzusehen.

Wie aus Figur 2 hervorgeht, erweist es sich als vorteilhaft, die Stollen 7 im Fersenbereich des Sohlenkörpers 1 im Hinblick auf ihre Aufstandsflächen größer zu bemessen als im mittleren und vorderen Bereich des Sohlenkörpers 1. Diese zusätzliche Abfederung im Anrollbereich, welcher bei der Abrollbewegung der Gehsohle am stärksten belastet ist, bringt somit auch eine verstärkte Entlastung, d.h. Abfederung für den Fuß mit sich.

Aufgrund der lösbaren Befestigung der elastischen Auflage 5 mit zugehörigen Stollen 6 durch "Einknüpfen" ist es auch möglich, je nach Heilungsgrad Auflagen und Stollen mit unterschiedlichen Elastizitätsgraden zu verwenden, so daß eine optimale Anpassung an den Heilungsprozeß durchführbar ist.

## Patentansprüche

1. Gehsohle für einen der Ruhigstellung und/oder Stützung dienenden Unter- und/oder Oberschenkelverband, welche aus einem Sohlenkörper (1) besteht, der eine für den Fuß als Aufstandsfläche dienende Oberseite und eine als Lauffläche dienende Unterseite aufweist, und mit Durchtrittsöffnungen (4) für ein Befestigungsband oder dgl. oder für mehrere Befestigungsbänder oder dgl. ausgerüstet ist, dadurch gekennzeichnet, daß auf die Oberseite und/oder Unterseite des Sohlenkörpers (1) eine Auflage (5) aus elastischem Material aufgebracht ist, welche die Oberseite bzw. Unterseite vollständig oder abschnittsweise überdeckt.

2. Gehsohle nach Anspruch 1, dadurch gekennzeichnet, daß die Auflage (5) an dem Sohlenkörper (1) durch Kraftschluß und/oder Formschluß befestigt ist.

3. Gehsohle nach Anspruch 1, dadurch gekennzeichnet, daß die Auflage (5) Stollen (7) aufweist.

4. Gehsohle für einen der Ruhigstellung und/oder Stützung dienenden Unter- und/oder Oberschenkelverband, welche aus einem Sohlenkörper (1) besteht, der eine für den Fuß als Aufstandsfläche dienende Oberseite und eine als Lauffläche dienende Unterseite aufweist, und mit Durchtrittsöffnungen (4) für ein Befestigungsband oder dgl. oder für mehrere Befestigungsbänder oder dgl. ausgerüstet ist, vorzugsweise nach Anspruch 1, dadurch gekennzeichnet, daß der Sohlenkörper (1) eine oder mehrere Durchtrittsöffnungen (6) aufweist, welche von Stollen (7) durchsetzt sind.

5. Gehsohle nach Anspruch 4, dadurch gekennzeichnet, daß die Stollen (7) aus elastischem Material bestehen.

6. Gehsohle nach Anspruch 1 und einem der vorhergehenden Ansprüche, dadurch gekennzeichnet, daß die Auflage (5) und die Stollen (7) aus demselben Material oder ähnlich dämpfenden Materialien hergestellt sind.

7. Gehsohle nach Anspruch 1 und einem der vorhergehenden Ansprüche, dadurch gekennzeichnet, daß die Auflage (5) und die Stollen (7) einstückig ausgebildet sind.

8. Gehsohle nach Anspruch 1 und einem der vorhergehenden Ansprüche, dadurch gekennzeichnet, daß die Stollen (7) in die Durchtrittsöffnungen (6) des Sohlenkörpers (1) eingeknüpft sind.

9. Gehsohle nach Anspruch 1 und einem der vorhergehenden Ansprüche, dadurch gekennzeichnet, daß die Stollen (7) an die Auflage (5) angeschäumt sind.

10. Gehsohle nach Anspruch 1 und einem der vorhergehenden Ansprüche, dadurch gekennzeichnet, daß die Durchtrittsöffnungen (6) des Sohlenörpers (1) sich zu dessen Unterseite hin konisch erweitern.

11. Gehsohle nach Anspruch 1 und einem der vorhergehenden Ansprüche, dadurch gekennzeichnet, daß die Stollen (7) die Durchtrittsöffnungen (6) des Sohlenkörpers (1) über dessen Unterseite überragen.

12. Gehsohle nach Anspruch 1 und einem der vorhergehenden Ansprüche, dadurch gekennzeichnt, daß die Durchtrittsöffnungen (6) über die Länge und/oder Breite des Sohlenkörpers (1) gleichmäßig verteilt angeordnet sind.

13. Gehsohle nach Anspruch 1 und einem der vorhergehenden Ansprüche, dadurch gekennzeichnet, daß im Fersenbereich des Sohlenkörpers (1) die Stollen (7) im Hinblick auf ihre Aufstandsflächen größer bemessen sind als in den übrigen Bereichen des Sohlenkörpers.

14. Gehsohle nach Anspruch 1 oder einem der vorhergehenden Ansprüche, dadurch gekennzeichnet, daß Auflagen (5) unterschiedlicher Elastizität Verwendung finden.

15. Gehsohle nach Anspruch 1 oder einem der vorhergehenden Ansprüche, dadurch gekennzeichnet, daß Stollen (7) unterschiedlicher Elastizität Verwendung finden.
